# EUROPEAN PATENT SPECIFICATION

(11) **EP 1 340 544 B1**
(45) Date of publication and mention of the grant of the patent: **14.12.2005**
(21) Application number: 02021881.4
(22) Date of filing: 30.09.2002
(51) Int. Cl.: B01L 3/00

(54) **Biochip including carbon nanotubes and method for sample separation using the same**
Biochip mit Kohlenstoff-Nanoröhrchen sowie Verfahren zur Trennung von Proben unter Verwendung dieses Biochips
Biochip avec nanotubes de carbone et procede de separation d' echantillons utilisant ce biochip

(30) Priority: 20.12.2001 KR 2001081780
(43) Date of publication of application: 03.09.2003
(73) Proprietor: SAMSUNG ELECTRONICS CO., LTD., Suwon-City, Kyungki-do (KR)
(72) Inventor: Kang, Seonhg-ho, Siheung-si, Gyeonggi-do (KR); Pak, Yukeun Eugene, Bundang-gu, Seongnam-si, Gyeonggi-do (KR); Choi, Won-bong, Kiheung-eub, Yongin-si, Gyeonggi-do (KR)
(74) Representative: Grünecker, Kinkeldey, Stockmair & Schwanhäusser Anwaltssozietät

(56) References cited:
- US-A- 5 648 569
- US-B1- 6 183 714
- ANALYTICAL BIOCHEMISTRY, vol. 257, 1998, pages 95-100, XP002229814

## Description

### BACKGROUND OF THE INVENTION

### 1. Field of the Invention

The present invention relates to a separation or filtration method, more particularly, to a biochip having a sample separation function and a method of separation or filtration of a target material from a sample in a biochip.

### 2. Description of the Related Art

Fusion of Biotechnology, nano-technology and MEMS(Micro Electro Mechanical System) technology created a new technical field called 'Biochip', and recently array-type DNA chips and protein chips are in an early commercializing phase. Moreover, the concept of Lab-on-a-chip (LOC) is introduced to develop DNA-LOC and protein-LOC, which integrate several functions such as sample pre-treatment, derivatization, separation, detection, analysis etc. in one chip, and thus are able to directly use various samples such as a natural sample, drug, food, medicine etc. as well as substantial biological samples such as blood, urine, cell, saliva (See: Stuart F. Brown, Fortune, Oct. 11. 1999. Good-bye Test Tubes, Hello Labs-on-a-Chip).

But, where an undesirable substance exists in a sample, it is difficult to obtain reliable data. For example, it is known that hemoglobin in red blood cell during PCR (Polymerase Chain Reaction) affects experimental data (See: McCusker, J., Dawson, M. T., Noone, D., Gannon, F., Smith, T. Nucleic Acids Res. 20, p 6747, 1992). Generally, such an undesirable substance is eliminated by pre-treatment employing centrifugation, a membrane which is made of various substances and has pores with various sizes, glass fiber, or various kinds of filtration devices. But, there are still many problems in directly applying these pre-treatment technologies to a micro-channel or chamber of LOC.

Recently, a method of filtrating and isolating cells from blood is disclosed, in which a filter is disposed in a chip using MEMS technology (See: Po Ki Yuen et al., Genome research, 11, p 405-412, 2001/ Peter Wilding et al., Analytical Biochemistry 257, p 95-100, 1998). Similarly, a method of separating a DNA having a desired size is disclosed (See: Oligica Bakalin et al., Anal. Chem., in press, 2001). But, these methods have limitations in that it is difficult to make spaces with nanometer size uniformly in a chip.

Carbon nano-tube is now a major component in miniaturized cathode-ray tubes (See: Nature 414, p 142-144, Nov 8, 2001), and is applied to variety of technical fields. For example, Lieber etc. disclosed a nanometer-scale microscopy probes using a carbon-based nano-tube (See: USP 6159742, Charles M. Lieber, Stanislaus S. Wong, Adam T. Woolley, Ernesto Joselevich. NANOMETER-SCALE MICROSCOPY PROBES). Eklund etc. made stable iodine-doped carbon nanotube or metallic nanometer-scale fiber by doping carbon nano-tube with iodine (See: USP 6139919,2000, METALLIC NANOSCALE FIBERS FROM STABLE IODINE-DOPED CARBON NANOTUBES), and Massey etc. made electrochemiluminescent ruthenium complexes using functional group biomolecule-modified nanotube (See: USP 5866434, 1999, Richard J. Massey et al., GRAPHITIC NANOTUBES IN LUMINESCEN ASSAYS).

But, it has not been reported that carbon nanotube is used to separate or filtrate a sample in a biochip.

### SUMMARY OF THE INVENTION

Therefore, the object of the present invention is to provide a biochip, which can be used in separation or filtration of a target substance contained in various kinds of samples, so as to prevent sample contamination or errors of the experimental data.

The other object of the present invention is to provide a method of separation or filtration of a target substance contained in various kinds of samples in the biochip; thus sample contamination or errors of the experimental data can be prevented.

Another object of the present invention is to provide a method of separation or filtration of a target substance contained in various kinds of samples by employing a plurality of carbon nanotubes disposed in an array type in a channel to have various intervals between adjacent carbon nanotubes.

To accomplish the first object, the present invention provides a biochip comprising;
a substrate;
a sample loading portion placed on the substrate;
a channel formed in the substrate and being in fluid communication with the sample loading portion; and
a plurality of carbon nanotubes arrayed in predetermined intervals in the channel.

To accomplish the second object, the present invention provides a method of separation or filtration of a target substance contained in a sample in a biochip comprising a substrate; a sample loading portion placed on the substrate; a channel in fluid communication with the sample loading portion; and carbon nanotubes arrayed in predetermined intervals in the channel,
the method comprising;
loading a sample including a target substance on the sample loading portion;
flowing the sample through the channel; and
separating the target substance included in the sample selectively according to the intervals between the carbon nanotubes.

To accomplish the third object, the present invention provides a method of separation or filtration of a target substance contained in a sample comprising;
disposing a plurality of carbon nanotubes in an array type in a channel to have various intervals between adjacent carbon nanotubes;
loading a sample containing a target substance on the channel; and
separating the target substance by flowing the sample through the channel.

### BRIEF DESCRIPTION OF THE DRAWINGS

The above objects and advantages of the present invention will become more apparent by describing in detail preferred embodiments thereof with reference to the attached drawings in which:
FIG. 1A - 1D are schematic cross sectional views illustrating a process for preparing carbon nanotubes on various substrates.
FIG. 2 is a schematic view illustrating separation and filtration of biological samples using carbon nanotubes.
FIG. 3 is a top view of carbon nanotube filters disposed in channels or chambers of various shapes.
FIG. 4 is a schematic view illustrating a process of separating white blood cells from a whole blood using multiple carbon nanotubes arrayed with various intervals in channels.

### DETAILED DESCRIPTION OF THE INVENTION

In the present invention, varieties of substances contained in a sample can be separated or filtrated selectively while passing through channels or chambers, each of channels or chambers including a plurality of carbon nanotubes. Specifically, according to varieties of intervals between adjacent carbon nanotubes, the substances can be separated.

The biochip of the present invention comprises a substrate, which has a sample loading portion and a channel. The channel is in fluid communication with the sample loading portion, and a plurality of carbon nanotubes are arrayed with predetermined intervals in the channel.

The substrate can be made from various materials such as silicon, melted silica, glass, and plastic.

Preferably, the channel of the biochip includes a plurality of unit channels.

The intervals between the carbon nanotubes in the biochip can be varied depending on a diameter of a target substance to be separated. Preferably, the intervals can be from several nanometers to several hundreds of micrometers.

Each of channels or chamber is of a length enough to accommodate a predetermined number of carbon nanotubes in array form with various intervals between the carbon nanotubes. Preferably, the length of the channel can be from several nanometers to several hundreds of millimeters.

The method of separation of a target substance contained in the sample in the biochip according to the present invention comprises the steps of loading the sample, flowing the sample, and separating the sample.

Loading the sample can be achieved by loading the sample including a target substance on the sample loading portion located on the surface of the biochip.

After loaded on the sample loading portion, the sample is made to flow through the channel by a force such as electric field, a pressure, a vacuumizing, an electromagnetic field, and a centrifugation force. Preferably, the flowing of the sample comprises opening and closing of the channels, that is, opening a portion of the plurality of the unit channels extended in a sample flow direction and closing the remaining unit channel(s). Then, the sample is flowed through the opened unit channel(s).

Finally, the selective separation of the target substance included in the sample can be achieved according to the intervals between the carbon nanotubes.

Preferably, the target substance includes biological molecules, and more preferably, the target substance is cells, nucleic acids, DNAs, proteins, peptides, polysaccharides, hormones, lipids, carbohydrates, or receptors.

Also provided is a general method of separating a target substance contained in a sample. It comprises steps of disposing a plurality of carbon nanotubes in an array type in a channel to have various intervals between adjacent carbon nanotubes, loading a sample containing a target substance into the channel, and separating the target substance by flowing the sample through the channel.

The invention is further described by the following examples. The examples are only for the purposes of illustration. It should be understood that the invention is not limited to the specific details of the examples.

### Example 1: Preparation of carbon nanotubes

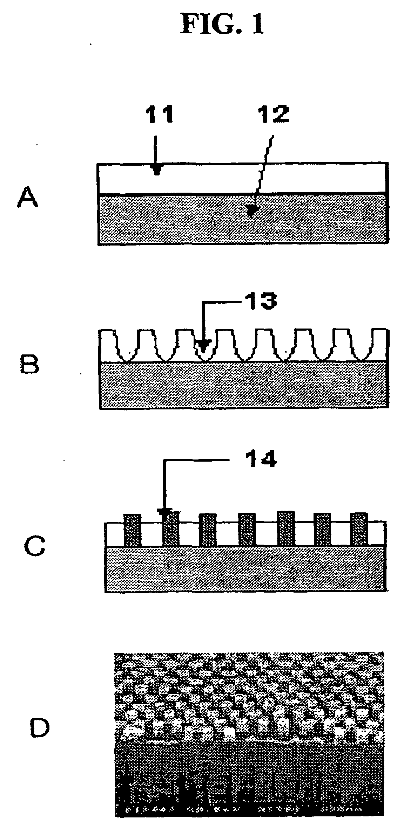

FIG. 1 shows a process of preparing carbon nanotubes on a substrate of a biochip.

Referring to FIG.1, channels or chambers each having a length of from nanometers to millimeters are prepared using various substances such as silicon, melted silica, glass, and plastic as a chip base substrate. In the channels or chambers, a plurality of carbon nanotubes each having a length of from nanometers to micrometers are prepared and arrayed in predetermined intervals.

More particularly, Al layer (11) was deposited on the substrate (12) as shown in FIG.1A. Then, as shown in FIG.1 B, Al layer was oxidized using anodic aluminum oxidation to form channels or chambers (13) each having a length of from tens of nanometers to hundreds of millimeters. Then, as shown in FIG. 1C, gases such as C₂H₂, and CH₄ are injected to prepare carbon nanotubes (14).

The diameters of the carbon nanotubes and the intervals between them may be controlled by a voltage applied and an oxidizer used in the anodic aluminum oxidation.

FIG. 1D is the photograph of the carbon nanotubes formed horizontally.

### Example 2: Separation or filtration of biological samples using carbon nanotubes inside the channels

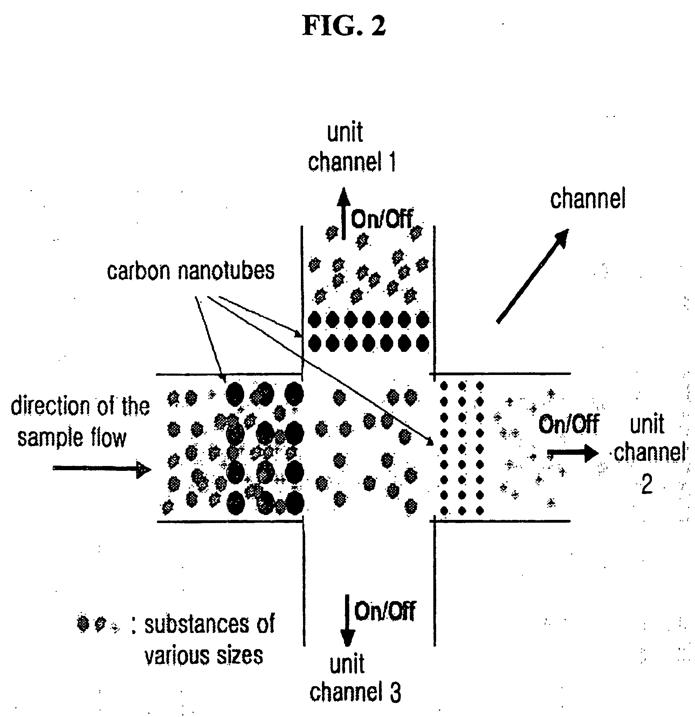

FIG.2 illustrates that one or more channels or chambers, each of which includes a plurality of carbon nanotubes arrayed with various intervals, can be used not only as a filtration device but also as a separation device. Various substances can be separated or filtrated by applying a force such as electric field, a pressure, a vacuumizing, an electromagnetic field, and a centrifugation force, to the channel so as to cause a fluid sample containing the various substances to flow.

Referring to FIG.2, to separate or filtrate a target substance in sample, the carbon nanotubes, which have different sizes and array patterns in each unit channels, are disposed. Then, the sample may be moved in one direction by opening a portion of the plurality of the unit channels extended in a sample flow direction and closing the remaining unit channel(s). (A conventional method such as using switch valve can be used for opening and closing the channels.) By the opening and closing of the unit channel(s), the intermediate sized samples, which have been filtrated through one unit channel, may be moved to the other desired unit channel. For example, after collecting the samples in central area by opening the desired unit channel 2 and closing the other two unit channels 1, 3, the collected samples in the central area, which are too large to pass through the carbon nanotubes in unit channel 2, can now be moved to the unit channel 1 by closing the unit channels 2, 3 and opening the unit channel 1.

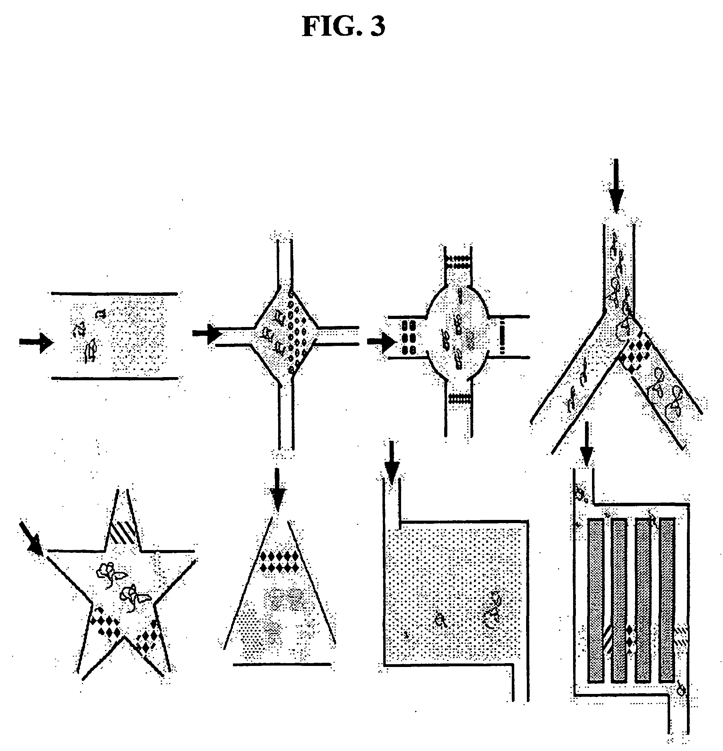

FIG.3 shows channels or chambers of various shapes in which carbon nanotubes are formed. In the figure, arrows indicate the direction of sample injection.

Referring to FIG.3, the channels or the chambers, which have various shapes, can be prepared, and the carbon nanotubes can be arrayed in appropriate positions of the channels or the chambers.

### Example 3: Separation of white blood cells from whole blood

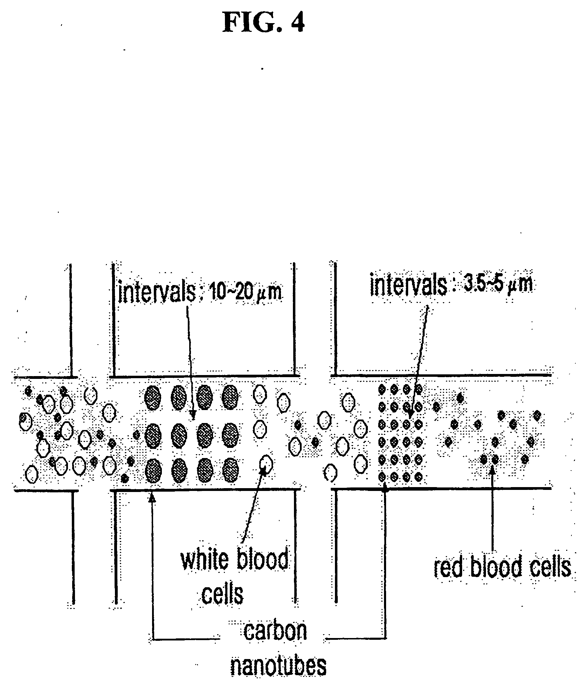

FIG.4 shows schematic diagram of a process for separating white blood cells from whole blood in channels on a chip. The white blood cells can pass through the carbon nanotubes in the channel when the intervals between the carbon nanotubes are about 10 to 20 micrometers. But, when the intervals between the carbon nanotubes are about 3.5 to 5 micrometers, the white blood cells cannot pass through the carbon nanotubes, and only red blood cells can pass through and flow to downstream of the channels.

As described above, according to the present invention, substances having various sizes can be selectively separated or filtrated, and carbon nanotubes can be formed in channels or chambers of various geometrical shapes. Therefore, high quality biochips, which have various designs can be provided.

As a result, carbon nanotubes of different sizes may be prepared per each of channels or chambers, thus comprehensive high-throughput carbon nanotube filters for separating or filtrating samples according to their sizes may be prepared. And it can be applied to Lab-on-a-Chip, since it can prevent sample contamination or errors of the experimental data.

## Claims

1. A biochip comprising;
a substrate (12);
a sample loading portion placed on the substrate;
a channel (13) formed in the substrate and being in fluid communication with the sample loading portion; and
a plurality of carbon nanotubes (14) arrayed with predetermined intervals in the channel.

2. The biochip according to claim 1, wherein the channel includes a plurality of unit channels.

3. The biochip according to claim 1 or 2, wherein the intervals between the carbon nanotubes are from several nanometers to several hundreds of micrometers.

4. The biochip according to any claims 1 to 3, wherein the intervals between the carbon nanotubes are from several nanometers to several tens of micrometers and preferably are larger than a lower limit of 1nm, 5nm, 10nm, 50nm or 100nm and are preferably smaller than an upper limit of 1 µm, 5µm, 10µm, 50µm or 100µm.

5. A biochip according to any claims 1 to 4, wherein the length of the channel is from several micrometers to several hundreds of millimeters.

6. The biochip according to any claims 1 to 5, wherein the length of the channel is from several micrometers to several tens of millimeters and preferably is larger than a lower limit of 1µm, 5µm, 10µm, 50µm or 100µm and preferably is smaller than an upper limit of 1mm, 5mm, 10mm, 50mm or 100mm.

7. A method of separation of a target substance contained in a sample in a biochip comprising a substrate;
a sample loading portion placed on the substrate;
a channel in fluid communication with the sample loading portion;
and carbon nanotubes arrayed with predetermined intervals in the channel,
the method comprising,
loading a sample including a target substance on the sample loading portion;
flowing the sample through the channel; and
separating the target substance included in the sample selectively according to the intervals between the carbon nanotubes.

8. The method according to claim 7, wherein the channel includes a plurality of unit channels, the flowing the sample through the channel comprises opening a portion of the plurality of the unit channels extended in a sample flow direction and closing the remaining unit channel(s) and flowing the sample through the opened unit channel(s).

9. The method according to claim 7 or 8, wherein the target substance includes biological molecules.

10. The method according to any claims 7 to 9, wherein the target substance is cells, nucleic acids, DNAs, proteins, peptides, polysaccharides, hormones, lipids, carbohydrates, or receptors.

11. A method of separation of target substance contained in a sample comprising;
disposing a plurality of carbon nanotubes in an array type in a channel to have various intervals between adjacent carbon nanotubes;
loading a sample containing a target substance on the channel; and
separating the target substance by flowing the sample through the channel.

## Patentansprüche

1. Biochip, der umfasst:
ein Substrat (12);
einen Proben-Bestückungsabschnitt, der auf dem Substrat angeordnet ist;
einen Kanal (13), der in dem Substrat ausgebildet ist und mit dem Proben-Bestückungsabschnitt in Fluidverbindung steht; und
eine Vielzahl von Kohlenstoff-Nanoröhrchen (14), die mit vorgegebenen Abständen in dem Kanal angeordnet sind.

2. Biochip nach Anspruch 1, wobei der Kanal eine Vielzahl von Einzelkanälen enthält.

3. Biochip nach Anspruch 1 oder 2, wobei die Abstände zwischen den Kohlenstoff-Nanoröhrchen von mehreren Nanometern bis zu mehreren hundert Nanometern reichen.

4. Biochip nach einem der Ansprüche 1 bis 3, wobei die Abstände zwischen den Kohlenstoff-Nanoröhrchen von mehreren Nanometer bis zu mehreren zehn Nanometern reichen und vorzugsweise größer sind als ein oberer Grenzwert von 1 nm, 5 nm, 10 nm, 50 nm oder 100 nm und vorzugsweise kleiner sind als ein oberer Grenzwert von 1 µm, 5 µm, 10 µm, 50 µm oder 100 µm.

5. Biochip nach einem der Ansprüche 1 bis 4, wobei die Länge des Kanals von mehreren Mikrometern bis zu mehreren hundert Millimetern reicht.

6. Biochip nach einem der Ansprüche 1 bis 5, wobei die Länge des Kanals von mehreren Mikrometern bis zu mehreren zehn Millimetern reicht und vorzugsweise größer ist als ein unterer Grenzwert von 1 µm, 5µm, 10µm, 50µm oder 100µm, und vorzugsweise kleiner ist als ein oberer Grenzwert von 1 mm, 5 mm, 10 mm, 50 mm oder 100 mm.

7. Verfahren zum Abscheiden einer Zielsubstanz, die in einer Probe in einem Biochip enthalten ist, der umfasst:
ein Substrat
einen Proben-Bestückungsabschnitt, der auf dem Substrat angeordnet ist;
einen Kanal, der in Fluidverbindung mit dem Proben-Bestückungsabschnitt steht;
und Kohlenstoff-Nanoröhrchen, die mit vorgegebenen Abständen in dem Kanal angeordnet sind,
wobei das Verfahren umfasst:
Bestücken des Proben-Bestückungsabschnitts mit einer Probe, die eine Zielsubstanz enthält;
Leiten der Probe durch den Kanal; und
selektives Abscheiden der Zielsubstanz, die in der Probe enthalten ist, entsprechend den Abständen zwischen den Kohlenstoff-Nanoröhrchen.

8. Verfahren nach Anspruch 7, wobei der Kanal eine Vielzahl von Einzelkanälen enthält, das Leiten der Probe durch den Kanal das Öffnen eines Teils der Vielzahl der Einzelkanäle, der sich in einer Proben-Fließrichtung erstreckt, und das Schließen des/der restlichen Einzelkanals/-kanäle sowie das Leiten der Probe durch den/die geöffneten Einzelkanal/-kanäle umfasst.

9. Verfahren nach Anspruch 7 oder 8, wobei die Zielsubstanz biologische Moleküle enthält.

10. Verfahren nach einem der Ansprüche 7 bis 9, wobei es sich bei der Zielsubstanz um Zellen, Nukleinsäuren, DNA, Proteine, Peptide, Polysaccharide, Hormone, Lipide, Kohlenwasserstoff oder Rezeptoren handelt.

11. Verfahren zum Abscheiden einer Zielsubstanz, die in einer Probe enthalten ist, das umfasst:
Anordnen einer Vielzahl von Kohlenstoff-Nanoröhrchen in einer Anordnung in einem Kanal, so dass verschiedene Abstände zwischen benachbarten Kohlenstoff-Nanoröhrchen vorhanden sind;
Bestücken des Kanals mit einer Probe, die eine Zielsubstanz enthält; und
Abscheiden der Zielsubstanz, indem die Probe durch den Kanal geleitet wird.

## Revendications

1. Biopuce comprenant :
un substrat (12);
une partie de chargement d'échantillon placée sur le substrat;
un canal (13) formé dans le substrat et étant en communication de fluide avec la partie de chargement d'échantillon; et
une multiplicité de nanotubes en carbone (14) disposés avec des intervalles prédéterminés dans le canal.

2. Biopuce selon la revendication 1, dans laquelle le canal comprend une multiplicité de canaux élémentaires.

3. Biopuce selon la revendication 1 ou 2, dans laquelle les intervalles entre les nanotubes en carbone vont de plusieurs nanomètres à plusieurs centaines de micromètres.

4. Biopuce selon l'une quelconque des revendications 1 à 3, dans laquelle les intervalles entre les nanotubes en carbone vont de plusieurs nanomètres à plusieurs dizaines de micromètres et sont de préférence plus grands qu'une limite inférieure de 1 nm, 5 nm, 10 nm, 50 nm ou 100 nm et sont de préférence plus petits qu'une limite supérieure de 1 µm, 5 µm, 10 µm, 50 µm ou 100 µm.

5. Biopuce selon l'une quelconque des revendications 1 à 4, dans laquelle la longueur du canal va de plusieurs micromètres à plusieurs centaines de millimètres.

6. Biopuce selon l'une quelconque des revendications 1 à 5, dans laquelle la longueur du canal va de plusieurs micromètres à plusieurs dizaines de millimètres et est de préférence plus grande qu'une limite inférieure de 1 µm, 5 µm, 10 µm, 50 µm ou 100 µm et est de préférence plus petite qu'une limite supérieure de 1 mm, 5 mm, 10 mm, 50 mm ou 100 mm.

7. Procédé de séparation d'une substance cible contenue dans un échantillon dans une biopuce comprenant
un substrat;
une partie de chargement d'échantillon placée sur le substrat;
un canal en communication de fluide avec la partie de chargement d'échantillon;
et des nanotubes en carbone disposés avec des intervalles prédéterminés dans le canal;
le procédé comprenant les opérations consistant à
charger sur la partie de chargement d'échantillon un échantillon incluant une substance cible;
faire circuler l'échantillon à travers le canal; et
séparer sélectivement la substance cible incluse dans l'échantillon, conformément aux intervalles entre les nanotubes en carbone.

8. Procédé selon la revendication 7, dans lequel le canal comprend une multiplicité de canaux élémentaires, la circulation de l'échantillon à travers le canal comprend l'ouverture d'une partie de la multiplicité des canaux élémentaires s'étendant dans une direction de circulation d'échantillon et la fermeture du ou des canaux élémentaires restants, et la circulation de l'échantillon à travers le ou les canaux élémentaires ouverts.

9. Procédé selon la revendication 7 ou 8, dans lequel la substance cible comprend des molécules biologiques.

10. Procédé selon l'une quelconque des revendications 7 à 9, dans lequel la substance cible consiste en cellules, acides nucléiques, ADN, protéines, peptides, polysaccharides, hormones, lipides, glucides ou récepteurs.

11. Procédé de séparation d'une substance cible contenue dans un échantillon, comprenant les opérations consistant à :
disposer une multiplicité de nanotubes en carbone en une configuration en réseau dans un canal de façon à avoir divers intervalles entre des nanotubes en carbone adjacents;
charger sur le canal un échantillon contenant une substance cible; et
séparer la substance cible en faisant circuler l'échantillon à travers le canal.
